# EUROPÄISCHE PATENTSCHRIFT

(11) **EP 2 435 104 B1**
(45) Veröffentlichungstag und Bekanntmachung des Hinweises auf die Patenterteilung: **04.11.2015**
(21) Anmeldenummer: 10726927.6
(22) Anmeldetag: 27.05.2010
(51) Int. Cl.: A61M 1/02, A61J 3/00

(54) **VORRICHTUNG UND VERFAHREN ZUR HERSTELLUNG VON BLUTPRODUKTEN**
DEVICE AND METHOD FOR PRODUCING BLOOD PRODUCTS
DISPOSITIF ET PROCÉDÉ DE PRÉPARATION DE PRODUITS SANGUINS

(30) Priorität: 27.05.2009 DE 102009022793
(43) Veröffentlichungstag der Anmeldung: 04.04.2012
(73) Patentinhaber: Justus-Liebig-Universität Gießen, 35390 Gießen (DE)
(72) Erfinder: HACKSTEIN, Holger, 35398 Gießen (DE); BEIN, Gregor, 35394 Gießen (DE); MISTEREK, Joachim, 35435 Wettenberg (DE)
(74) Vertreter: Patentanwälte Olbricht Buchhold Keulertz
(86) Internationale Anmeldenummer: PCT/EP2010/057344
(87) Internationale Veröffentlichungsnummer: WO 2010/136535

(56) Entgegenhaltungen:
- DE-A1- 2 654 725
- DE-B- 1 123 800
- US-A- 3 870 042
- US-A1- 2004 078 021
- DATABASE WPI Week 200866 Thomson Scientific, London, GB; AN 2008-L21463 XP002601893 & CN 201 058 029 Y (HE W) 14. Mai 2008 (2008-05-14)

## Beschreibung

Die Erfindung betrifft eine Vorrichtung zur Entnahme von Blut und Herstellung von Blutprodukten daraus und deren Lagerung und Transport, umfassend Entnahmemittel und mindestens ein Sammel- und Trennmittel sowie ein Verfahren zur Entnahme von Blut und Herstellung von Blutbestandteilen daraus in einer solchen Vorrichtung.

### Stand der Technik

Unter "Blutprodukte" sind zu verstehen: Blutzubereitungen im Sinne des Arzneimittelgesetzes, Sera aus Blut im Sinne des Arzneimittelgesetzes sowie Blutbestandteile oder Blutkomponenten oder Blutzubereitungen, die zur Herstellung von Wirkstoffen oder Arzneimitteln bestimmt sind.

Unter "Blutspender" ist zu verstehen: Ein Mensch oder ein Tier, bevorzugt ein Wirbeltier, dem eine bestimmte Menge an Blut oder Blutbestandteilen entnommen wird, um als Wirkstoff oder Arzneimittel eingesetzt zu werden, oder zur Herstellung von Wirkstoffen oder Arzneimitteln oder anderen Produkten, welche zur Anwendung bei Menschen oder Tieren bestimmt sind. Unter "Blut" bzw. "Vollblut" ist zu verstehen: Sämtliche nativen Bestandteile enthaltendes Blut eines Menschen oder eines Tieres, bevorzugt eines Wirbeltieres, nach einer Blutspende.

Unter "Blutbestandteile" sind zu verstehen: Alle aus Blut bzw. Vollblut gewinnbaren Bestandteile, die mit manuellen oder maschinellen Auftrennungsverfahren gewonnen oder direkt durch Hämapherese hergestellt werden.

Unter "Hämapherese" ist zu verstehen: Die Auftrennung von Blut bzw. Vollblut in verschiedene Blutbestandteile durch die Anwendung von Zellseparatoren mit extrakorporalem Kreislauf unmittelbar am Blutspender, wobei die nicht benötigten Blutbestandteile dem Spender unmittelbar wieder zugeführt werden. Unter "Blutserum" bzw. "Serum" ist zu verstehen: Jener Teil des Blutes bzw. Vollblutes, der nach Ablauf der Blutgerinnung als flüssiger, proteinreicher aber zellarmer Überstand verbleibt.

Unter "Eigenserum" bzw. "Eigenblutserum" ist zu verstehen: Dem jeweiligen Patienten eigenes Blutserum, welches nach einer autologen Blutspende gewonnen wurde.

Unter "Augentropfen" *(oculoguttae)* sind zu verstehen: Eine Darreichungsform von Arzneimitteln zur Anwendung am oder im Auge.

Unter "Eigenserumaugentropfen" sind zu verstehen: Augentropfen, die aus Eigenserum hergestellt werden.

Unter "*predonation sampling*" ist zu verstehen: Abtrennung von mindestens 15 ml des initialen Blutvolumens von der Blutspende zur Verminderung der bakteriellen Kontamination des Blutes bzw. Vollblutes im Zuge der Blutentnahme. Üblicherweise wird das *predonation sampling* mittels eines *predonation sampling bags* durchgeführt.

Unter "GMP" ist zu verstehen: *good manufacturing practise,* gute Herstellungspraxis bei der Herstellung von Arzneimitteln, Medizinprodukten, Wirkstoffen sowie Lebensmitteln und Futtermitteln.

Dem Fachmann ist die Durchführung einer gesetzes- und GMP-konformen Blutspende, sowohl als Vollblutspende als auch durch Hämapherese bekannt (siehe beispielsweise Transfusionsgesetz TFG inklusive Richtlinien oder Annex 1 der EU Guidelines für *good manufacturing practice*). Ebenso ist die gesetzes- und GMP-konforme Herstellung von Blutbestandteilen und Blutprodukten bekannt. Dasselbe gilt für die Herstellung von Augentropfen. Dies gilt sowohl für die Humanmedizin als auch die Veterinärmedizin.

Ein wesentlicher Punkt bei der Durchführung einer Blutspende und der Herstellung von Blutprodukten ist die Wahl eines geeigneten Behälters. Im Stand der Technik sind zwar zahlreiche Behälter zur Aufnahme von Blut, wie aus DE 1 065 138 A1 (Fa. B. Braun, AT 15.6.1956) oder US 5 224 937 (NPBI, AT 21.06.1991) oder auch ein System zur Blutentnahme und Blutsammlung bekannt, so z.B. aus der DE 1 123 800 (Fenwal Laboratories, Inc., AT 12.2.1957).

Diese weisen aber keine geeigneten Verabreichungsmittel auf, welche die innerhalb der Vorrichtung aus dem Blutprodukt erzeugten Arzneimittel für einen Patienten direkt, d.h. ohne Hilfsmittel, applizierbar gestalten oder es handelt sich nicht um geschlossene Systeme, in dem Sinne, dass das System, bis auf den Schritt der Entnahme von Blut am Blutspender und den Schritt der Applikation am Patienten, nicht geöffnet wird.

Die US 3,870,042 zeigt ein System zur Blutentnahme mit einer Nadel zur Speicherung von Blut in einem ersten flexiblen Beutel, in dem, beispielsweise durch Zentrifugieren, Blutplasma gewonnen wird. In mindestens einem weiteren Behälter wird dann aus dem Plasma, beispielsweise über eine Kältefällung, der Gerinnungsfaktor VIII präpariert. Das auf diese Art gewonnene Blutpräparat wird dann in einem Speicherbehälter gesammelt. Aus diesem Speicherbehälter kann das Faktor VIII Präparat dann über eine Injektionsnadel in eine Vene des Patienten injiziert werden. Die danach vorgeschlagene Vorrichtung ist insbesondere nicht für eine Applikation von Serum am Auge eines Patienten geeignet, sondern ist für eine Injektion in die Blutbahn des Patienten vorgesehen.

Die CN 201058029 Y beschreibt eine Vorrichtung zur Gewinnung von Blutserumaugentropfen mit einer Blutsammelflasche, einer Plasmaverdünnungsflasche und damit verbundenen Augentropfenfläschchen. Ein Entnahmemittel zur Gewinnung von Blut ist demnach nicht vorgesehen.

Damit besteht im Stand der Technik ein Mangel an geeigneten Vorrichtungen zur einfachen gesetzes- und GMP-konformen Herstellung, Lagerung, Transport und vor allem Applikation von Blutprodukten, vor allem von aus Blut oder Blutserum hergestellten Augentropfen.

Nach Vorschrift des Europäischen Arzneibuchs (*Pharmacopoea Europaea, Ph. Eur.*) sind Augentropfen immer steril herzustellen. Als apothekenpflichtiges Medikament dürfen diese in Deutschland nur in Apotheken abgegeben werden. Augentropfen sind entweder in Einmaldosisbehältnissen aus Kunststoff oder Augentropffläschchen aus speziellem Glas (Braunglas Glasart I) erhältlich. Neben der industriellen Herstellung dürfen Augentropfen auch rezepturmäßig in der Apotheke nach Verordnung des Arztes hergestellt werden. Dafür ist allerdings ein Reinstraumlaboratorium erforderlich, welches in der Regel in Apotheken nicht vorhanden ist.

Ein besonderes Problem stellt derzeit die gesetzes- und GMP-konforme Herstellung, Lagerung, Transport und Applikation von Augentropfen aus Blutserum dar, welche, besonders als Eigenserumaugentropfen, eine wichtige Rolle in der Behandlung von Augenerkrankungen spielen. Typische Anwendungsgebiete für diese aus Eigenserum gewonnenen Augentropfen sind die Behandlung von Trockenheit und Reizung des Auges, das Glaukom, Bindehautentzündungen (Konjunktivitis) und Hornhautentzündungen (Keratitis).

Aber auch für andere ophthalmologische Zwecke wird mittlerweile Eigenserum erfolgreich eingesetzt. So wird -nach Angaben des Klinikums Rechts der Isar der TU München im Jahresbericht 2003- zur Versorgung von retinalen Lochbildungen an der Stelle des schärfsten Sehens (Makulaforamina) auch eine Klebung mit Eigenserum routinemäßig durchgeführt, d.h. Eigenserum wird als Kleber eingesetzt.

Weitere Anwendungsgebiete, in denen sich seit etwa 10 Jahren Augentropfen aus Serum von Spender selbst gewonnen (autolog oder auch Eigenserum genannt) oder von Fremdspendern (allogen oder auch Fremdserum genannt), bewährt hat, sind in "Eigenserum und alternative Blutprodukte zur Behandlung von Augenoberflächenerkrankungen" von G. Geerling et al. in "Der Ophthalmologe" 7, 2008, S. 623ff und S. 644ff, genannt und betreffen zahlreiche Augen- und Augenoberflächenerkrankungen.

Derzeit werden beispielsweise Eigenserumaugentropfen in England hergestellt, indem Eigenblutspenden an überregionale Reinstraumlaboratorien gesendet werden, dort zu Eigenserumaugentropfen verarbeitet, aliquotiert und abgefüllt werden und anschließend wieder an den behandelnden Arzt zurückgeschickt werden. Dieses Verfahren ist zeit- und kostenintensiv.
Eine Herstellung von Eigenserumaugentropfen in den Blutspendeeinrichtungen (z.B. Blutbank) selber ist bisher nicht möglich, da es dort meist an den gesetzlich erforderlichen Reinstraumlaboratorien mangelt.

### Aufgabe der Erfindung

Aufgabe der vorliegenden Erfindung ist es daher, die vorgenannten Nachteile im Stand der Technik zu beheben und eine Vorrichtung und ein Verfahren bereit zu stellen, in welcher und durch welches aus Blut eines Blutspenders, ohne die Notwendigkeit eines Reinstraumlaboratoriums, direkt innerhalb der Vorrichtung und unter fortgesetztem Verschluss dieser Vorrichtung (nach der Befüllung und bis zur Applikation) Arzneimittel, umfassend Blut oder Blutprodukte im weitesten Sinne, wie etwa Blutserum, herstellbar sind. Die Vorrichtung ist dabei so auszuführen, dass diese aus Blut oder Blutprodukten hergestellten Arzneimittel nachfolgend direkt aus Elementen der Vorrichtung und ohne weitere Hilfsmittel applizierbar sein sollen. Zusätzlich sollen die Vorrichtung und das Verfahren so beschaffen sein, dass sowohl durch eine Vollblutspende als auch durch Hämapherese das gewünschte Blutprodukt hergestellt werden kann. Die Vorrichtung und das Verfahren sollen dabei so ausgeführt sein, dass sie sowohl zum Einsatz bei menschlichen als auch bei tierischen Patienten geeignet sind.

### Lösung

Die gestellte Aufgabe wird erfindungsgemäß durch eine Vorrichtung gemäß Anspruch 1 gelöst. Danach umfasst die Vorrichtung ein Entnahmemittel und mindestens ein Sammel- und Trennmittel, dadurch gekennzeichnet, dass die Vorrichtung eine Vielzahl an Verabreichungsmitteln umfasst, wobei Entnahmemittel, Sammel- und Trennmittel und die Verabreichungsmittel flüssigkeitsdurchströmbar miteinander verbunden sind und jedes Verabreichungsmittel ein Öffnungsmittel aufweist

welches zur Öffnung durch einen Patienten oder eine andere Person ohne weitere Hilfsmittel geeignet ausgeführt ist. Bezüglich des Verfahrens wird die gestellte Aufgabe erfindungsgemäß durch den Gegenstand von Anspruch 14 gelöst.

Mit der in der vorliegenden Erfindung beschriebenen Vorrichtung und dem darin beschriebenen Verfahren lassen sich in kurzer Zeit, mit geringem Einsatz von Personal- und Sachkosten in jeder medizinischen Einrichtung mit einer Blut- oder Eigenblut-Spendeeinrichtung Blutprodukte wie beispielsweise Serum- oder Eigenserumaugentropfen oder ähnliche Arzneimittel aus Blut oder Blutkomponenten gesetzes- und GMP-konform herstellen, lagern, transportieren und damit für die Patienten verfügbar machen. Die gilt für Vollblutspenden ebenso wie für Hämapheresen, sowohl für menschliche als auch tierische Patienten.

Die Erfindung zeichnet sich dadurch aus, dass nach der Befüllung der Vorrichtung mit Blut (bevorzugt Eigenblut) keine Öffnung der Vorrichtung mehr erfolgt, welche als unsteril zu bezeichnen ist (außer bei der Applikation des durch das erfindungsgemäße Verfahren hergestellten Blutproduktes am Patienten, wie beispielsweise Eigenblutserum-Augentropfen beim Einträufeln auf das Auge). D.h. das erfindungsgemäße Verfahren ist so gestaltet, dass eine GMP- und gesetzeskonforme (etwa "Annex 1 der EU Guidelines für *good manufacturing practice*" *oder* "Gesetz zur Regelung des Transfusionswesens", Transfusionsgesetz TFG) Herstellung von Blutprodukten, wie beispielsweise solchen Augentropfen aus dem Blut von Fremdspendern oder Eigenspendern gewährleistet ist.

Die erfindungsgemäße Vorrichtung und das Verfahren erlauben somit erstmalig eine allen Gesetzesbestimmungen konforme direkte Herstellung von Eigenserumaugentropfen am Ort der Blutspende selber.

Eine bevorzugte Ausführung der Erfindung betrifft eine Vorrichtung und ein Verfahren zur Herstellung, Lagerung, Transport und Applikation von Blutprodukten (oder Blutkomponenten oder Blutbestandteilen oder Blutzubereitungen im Sinne des Arzneimittelgesetzes AMG und des Transfusionsgesetzes TFG) nach Entnahme von Blut, sowohl als Vollblutspende als auch durch Hämapherese, ohne die Notwendigkeit von Reinstraumlaboratorien. Insbesondere betrifft sie ein geschlossenes Blutentnahme- und Verarbeitungssystem, sowie ein System zur Lagerung, zum Transport und zur Applikation von Blutprodukten und ein entsprechendes Verfahren zur gesetzes- und GMP-konformen Gewinnung, Herstellung, Lagerung, Transport und Applikation von Blutprodukten ohne die Notwendigkeit von Reinstraumlaboratorien. In ganz besonderer Weise betrifft die vorliegende Erfindung die Herstellung, Lagerung, Transport und Applikation von fertig aliquotierten, direkt am Patienten anwendbaren Blutprodukten wie beispielsweise Eigenserumaugentropfen oder anderer Arzneimittel bestehend aus Blutprodukten ohne die Notwendigkeit von Reinstraumlaboratorien.

Eine bevorzugte Ausführung der Erfindung betrifft also insbesondere ein geschlossenes Blutentnahme- und Verarbeitungssystem zur Herstellung, Lagerung, Transport und Applikation von Eigenserumaugentropfen unter gesetzes- und GMP-konformen Bedingungen, für die kein Reinstraumlaboratorium notwendig ist.

Die Vorrichtung und das Verfahren sind für die Entnahme und Verarbeitung von Blut von Eigenblut- oder Fremdblutspendern, d.h. von autolog bzw. allogen gewonnenem Blut anwendbar. Dabei können für autolog bzw. allogen gewonnenes Blut ggf. weitere Mittel zur Sterilisation an oder in der Vorrichtung z.B. in Form von Flüssigkeiten innerhalb der Vorrichtung vorgesehen sein.
Die Vorrichtung und das Verfahren sind sowohl im Anschluss an eine Vollblutspende als auch nach einer Hämapherese einsetzbar.

Ebenso sind die Vorrichtung und das Verfahren für menschliche Patienten und tierische Patienten, in besonderer Weise für Wirbeltiere, gleichermaßen geeignet.

Damit beschreibt die Erfindung erstmalig die Möglichkeit, aus Eigen- oder Fremdblut oder deren Bestandteilen Arzneimittel anzufertigen, welche aliquotiert werden können und direkt am Patienten von dem Patienten selber oder einer anderen Person oder an einem Tier, bevorzugt an einem Wirbeltier, ohne weitere Hilfsmittel einsetzbar sind.

Dies ist besonders bei Patienten mit bestimmten Augenerkrankungen von großer Bedeutung, da die gesetzes- und GMP-konforme Herstellung von Eigenserumaugentropfen aufwändig ist, v.a. durch die Notwendigkeit von Reinstraumlaboratorien bei der Aliquotierung und Abfüllung der Eigenserumaugentropfen in geeignete Verabreichungsmittel.

Es ist dem Fachmann unmittelbar ersichtlich, dass die erfingungsgemäße Vorrichtung durch Verwendung von dafür geeigneten Materialien ausführbar ist.

### Beispiele:

Ausführungsbeispiele sind in den Figuren 1 bis 5 dargestellt: Zusätzlich ist ein Ausführungsbeispiel in der Figur 6 dargestellt.
**Fig.1** zeigt eine nicht erfindungsgemäße Vorrichtung in einem ersten Ausführungsbeispiel in einem Schnitt mit einem Entnahmemittel (1), einem Sammel- und Trennmittel (5), einer Zuführleitung (6) und einem Verabreichungsmittel (10), welches in diesem Ausführungsbeispiel mit einem Öffnungsmittel (11) und mit einer Vorrichtung für eine Markierung (12) versehen ist.
**Fig.2** zeigt ein zweites Ausführungsbeispiel -ebenfalls im Schnitt- mit einer Vielzahl von Verabreichungsmitteln (14), wobei das in Füllrichtung gesehene letzte Verabreichungsmittel als hinterstes Verabreichungsmittel (15) bezeichnet wird.
**Fig.3** zeigt ein drittes Ausführungsbeispiel -ebenfalls im Schnitt- mit einem Sammelmittel zur Durchführung des *predonation samplings* (2), dem sog. *predonation sampling bag,* einem zusätzlichen Sammel- und Trennmittel (7), einer weiterführenden Zuführleitung (8) und einem endständigen Aufnahmemittel (16).
**Fig.4** zeigt eine Vergrößerung der Vielzahl -ebenfalls im Schnitt- von Verabreichungsmitteln (14) mit Markierung der Füllung und der Füllrichtung.
**Fig.5** zeigt eine Vergrößerung der Vielzahl -ebenfalls im Schnitt- von Verabreichungsmitteln (14) nach Trennung der einzelnen Verabreichungsmittel voneinander und der Trennung von der mindestens einen Zuführleitung (6 bzw. 8) und vom endständigen Aufnahmemittel (16).
**Fig. 6** zeigt eine Vergrößerung der Vielzahl von Verabreichungsmitteln (14), welche unabhängig von den Entnahme-, Sammel- und Trennmittel dargestellt sind.

Die Abbildungen werden im Folgenden näher beschrieben:

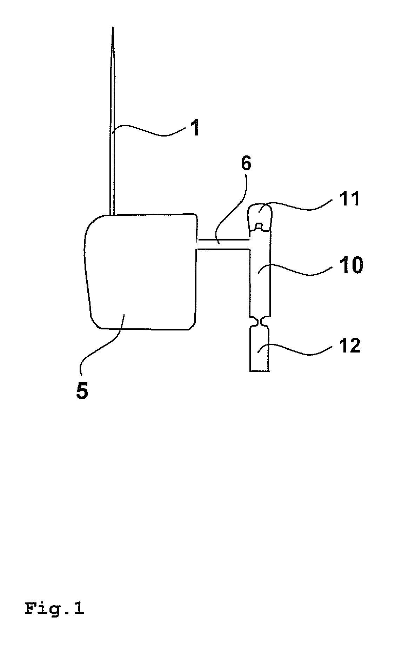

**Fig. 1** zeigt als einfachstes Ausführungsbeispiel die Anordnung der einzelnen Bestandteile der erfindungsgemäßen Vorrichtung gemäß Anspruch 1. Soweit dieses Ausführungsbeispiel nur ein einziges Verabreichungsmittel (10) aufweist, gehört es nicht zum Gegenstand der vorliegenden Erfindung. Das Entnahmemittel (1) führt das entnommene Blut zum mindestens einen Sammel- und Trennmittel (5), wo, sofern als Endprodukt Blutserum gewünscht ist, die Blutgerinnung stattfindet. Das als Folge der Blutgerinnung sich absetzende Blutserum wird aus dem mindestens einem Sammel- und Trennmittel (5) über die mindestens eine Zuführleitung (6) in das mindestens eine Verabreichungsmittel (10) überführt. Vorzugsweise ist das mindestens eine Verabreichungsmittel (10) mit einem Öffnungsmittel (11) versehen, welches es dem Verabreichenden ermöglicht, das Verabreichungsmittel (10) vor der Verwendung auf eine einfache Weise ohne weitere Hilfsmittel zu öffnen. Weiterhin ist vorzugsweise das mindestens eine Verabreichungsmittel (10) mit einer Vorrichtung für eine Markierung (12) versehen, die es ermöglicht, eine dauerhafte und unverwechselbare Kennzeichnung, beispielsweise eine fortlaufenden Seriennummer oder einen Barcode an dem Verabreichungsmittel (10) anzubringen.

Eine weitere vorteilhafte -nicht dargestellte- Ausführungsform sieht den Einsatz von weiteren Mitteln vor (wie beispielsweise ein Antikoagulans zur Gewinnung von Blutplasma, oder beispielsweise ein Verdünnungsmittel zur Verdünnung des gewonnenen Blutproduktes oder beispielsweise eine oder mehrere pharmazeutisch akzeptable Substanzen). Die Auswahl und die Anordnung dieser weiteren Mittel richten sich nach dem gewünschten Blutprodukt und sind dem Fachmann unmittelbar ersichtlich.

Ein weiteres besonders bevorzugtes -nicht graphisch dargestelltes- Ausführungsbeispiel zeigt die Vorsehung einer Absperrung, etwa in Form einer Schlinge, eines Knotens, einer Klemme, einer Kugel, eines Kegels, eines Ventils, eines verkapselten Abbrechteiles oder einer Knickbrechverbindung unmittelbar vor der Mündung des Entnahmemittels (1) in das mindestens eine Sammel- und Trennmittel (5). Zum Einlass des Blutes wird dann die Kugel, der Kegel, das verkapselte Abbrechteil oder die Knickbrechverbindung händisch in das mindestens eine Sammel- und Trennmittel (5) gedrückt, von wo diese nicht weiter gelangen können, da der Querschnitt der Zuführleitung (6) kleiner ausgeführt ist als der Querschnitt des Entnahmemittels (1). Eine ebensolche Absperrung ist vorteilhaft am Übergang vom mindestens einem Sammel- und Trennmittel (5) zur Zuführleitung (6) angebracht, um den unbeabsichtigten oder vorzeitigen Übertritt von Blut oder Blutbestandteilen aus dem mindestens einem Sammel- und Trennmittel (5) in die Zuführleitung (6) zu verhindern.

Es ist dem Fachmann unmittelbar ersichtlich, dass die erfindungsgemäße Vorrichtung durch Verwendung von geeigneten und für die Verwendung zur Sammlung, Lagerung, Transport und Applikation von Blut oder Blutprodukten zugelassenen Materialien ausführbar ist.

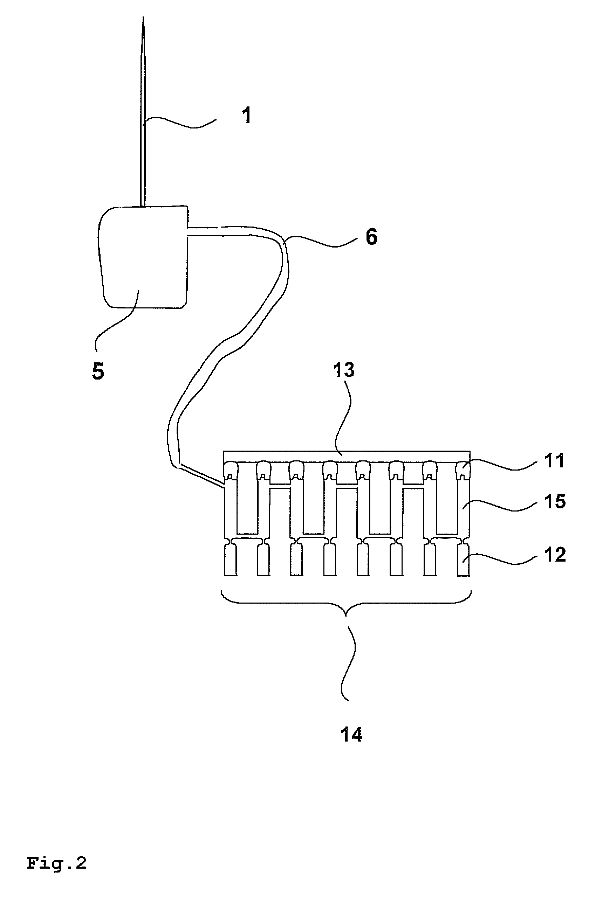

**Fig. 2** zeigt in einem vorteilhafteren Ausführungsbeispiel die Anordnung der einzelnen Bestandteile der erfindungsgemäßen Vorrichtung. Das Entnahmemittel (1) führt das entnommene Blut zum mindestens einen Sammel- und Trennmittel (5). Das gewünschte Blutprodukt wird aus dem mindestens einem Sammel- und Trennmittel (5) über die mindestens eine Zuführleitung (6) in eine beliebige Vielzahl von Verabreichungsmitteln (14) überführt. Die in den Figg. 2 bis 5 gezeigten acht Verabreichungsmittel sind als ein Beispiel zu verstehen und sollen keinesfalls die mögliche Anzahl der Verabreichungsmittel einschränken. Bei einer Vielzahl von Verabreichungsmittel sind diese erfindungsgemäß bis zum hintersten Verabreichungsmittel (15) so in einer Reihenfolge angeordnet, dass sie vollständig mit dem Blutprodukt gefüllt werden können, welches zur Anwendung bei den Patienten vorgesehen ist. Zusätzlich sind bei einer Vielzahl von Verabreichungsmittel diese erfindungsgemäß so mit einem Verbindungssteg (13) verbunden, dass sie eine Einheit bilden und einzeln entnommen werden können.

Auch bei einer Vielzahl von Verabreichungsmittel ist jedes einzelne von diesen vorzugsweise mit einem Öffnungsmittel (11) und einer Vorrichtung für eine Markierung (12) versehen.

Ein weiteres besonders bevorzugtes -nicht graphisch dargestelltes- Ausführungsbeispiel sieht zusätzlich noch folgende Modifikationen vor:
- Vorsehung einer Abzweigung (beispielsweise in Form eines Dreiwegehahnes oder eines Y-Stückes) am Entnahmemittel (1), um die ersten ca. 15 ml des Spenderblutes im Sinne des *predonation samplings* in einen abtrennbaren Teil der Vorrichtung zu führen, beispielsweise in ein Sammelmittel zur Durchführung des *predonation samplings* (2), bevor dann das weitere Blut in das mindestens eine Sammel- und Trennmittel (5) geleitet wird (siehe Richtlinien zum Transfusionsgesetz). Diese Abzweigung kann besonders vorteilhaft in Form eines sog. *predonation sampling bags* ausgeführt sein.
- parallele Anströmung der Vielzahl von Verabreichungsmittel (14) durch mindestens zwei Zuführleitungen, wodurch sowohl von unten als auch von oben die Vielzahl der Verabreichungsmittel (14) befüllt werden können.

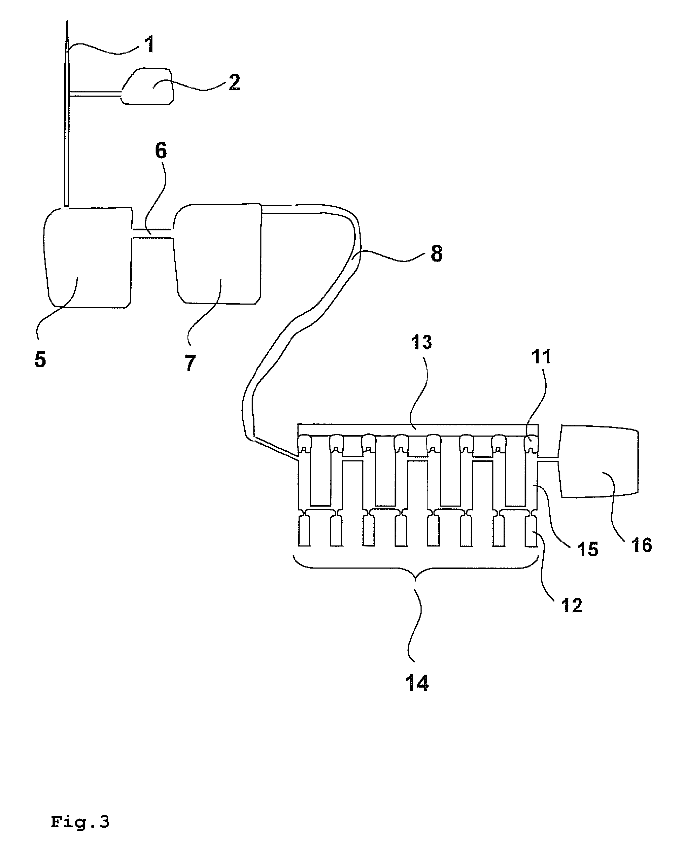

**Fig. 3** zeigt in einem noch vorteilhafteren Ausführungsbeispiel die Anordnung der einzelnen Bestandteile der erfindungsgemäßen Vorrichtung. Zusätzlich zu den bereits erwähnten Mitteln wird zwischen dem Sammel- und Trennmittel (5) und der Zuführleitung (6) mindestens ein zusätzliches Sammel- und Trennmittel (7) eingefügt, um die Trennung des Blutes in die gewünschten Blutprodukte zu verbessern. An dem Entnahmemittel (1) ist das Sammelmittel zur Durchführung des *predonation samplings* (2) angefügt, welches die Gewinnung einer Blutprobe für chemische, serologische, labordiagnostische und bakteriologische Untersuchungen ermöglicht, ohne die Sterilität der gesamten Vorrichtung zu gefährden. Besonders vorteilhaft ist die Vorsehung einer Absperrung, etwa in Form einer Schlinge, eines Knotens, einer Klemme, einer Kugel, eines Kegels, eines Ventils, eines verkapselten Abbrechteiles oder einer Knickbrechverbindung unmittelbar am Übergang von dem Entnahmemittel (1) zum Sammelmittel zur Durchführung des *predonation samplings* (2) (in der Zeichnung nicht gezeigt). Des Weiteren wird an das hinterste Verabreichungsmittel (15) ein endständiges Aufnahmemittel (16) angefügt, welches die Befüllung der Verabreichungsmittel erleichtert.

Ein weiteres besonders bevorzugtes -nicht graphisch dargestelltes- Ausführungsbeispiel sieht zusätzlich noch folgende Modifikationen vor:
- Vorsehung von mindestens einem Behälter zur Beimischung von Mitteln etwa zur Verdünnung des erzeugten Blutproduktes, zur Hemmung der Blutgerinnung oder zur Zuführung weiterer pharmazeutisch akzeptabler Substanzen.
- Vorsehung einer Vorrichtung, um mindestens einmal eine Flüssigkeitsunterbrechung im Sinne des Strömungsweges innerhalb der gesamten erfindungsgemäßen Vorrichtung zu aktivieren oder zu deaktivieren.
- Vorsehung von Dokumentationsmitteln jeweils vor und hinter jeder Trennstelle, um die Zuordbarkeit vom Blut im mindestens einem Sammel- und Trennmittel (5) zu dem mindestens einen Verabreichungsmittel (10 bzw. 14) zu gewährleisten.

- Vorsehung, dass die komplette Vorrichtung steril angeliefert wird.
- Vorsehung, dass die komplette Vorrichtung luftleer angeliefert wird.
- Vorsehung, dass die komplette Vorrichtung unter Druck von steriler Luft stehend angeliefert wird, so dass ein Eintritt von unsteriler Luft beim Prozess der Venenpunktion im Zuge der Blutentnahme unmöglich ist. In diesem Fall ist notwendigerweise ein endständiges Aufnahmemittel (16) für Luft am Ende des Strömungsweges vorzusehen.
- Vorsehung, die eine Verbringung des Blutes oder einzelner Blutprodukte durch eine Sterilfiltrationseinheit oder einen Leukozytenfilter oder sonstigen Zellfilter ermöglicht.

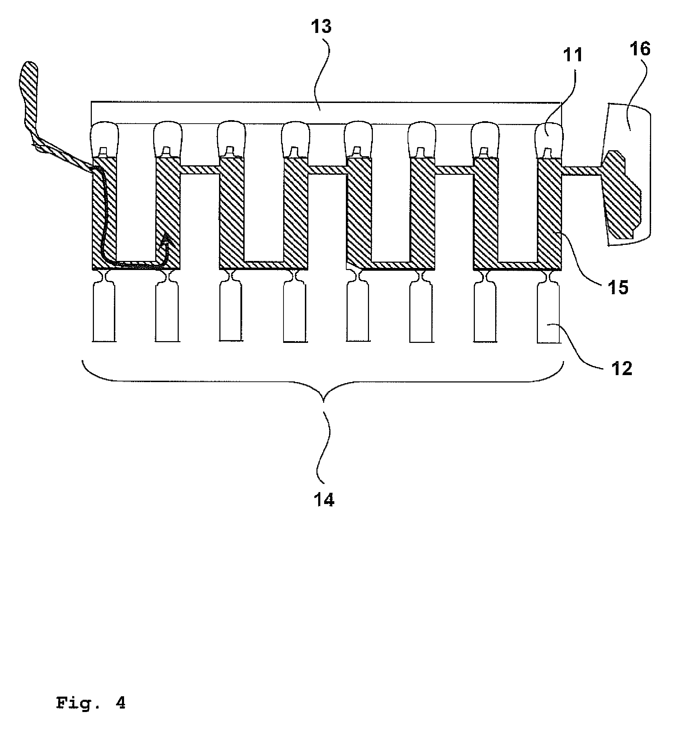

**Fig. 4** zeigt in einer vergrößerten Detailansicht die Vielzahl der Verabreichungsmittel (14) und schraffiert dargestellt die Füllung der Verabreichungsmittel mit dem gewünschten Blutprodukt. Der Pfeil zeigt die Fließrichtung des Blutproduktes bei der Füllung der Verabreichungsmittel (14) mittels des erfindungsgemäßen Verfahrens an. Um eine vollständige Füllung aller Verabreichungsmittel zu erreichen, wird eine solche Menge des gewünschten Blutproduktes in die Verabreichungsmittel überführt, dass der Flüssigkeitsstrom bis in das endständige Aufnahmemittel (16) gelangt.

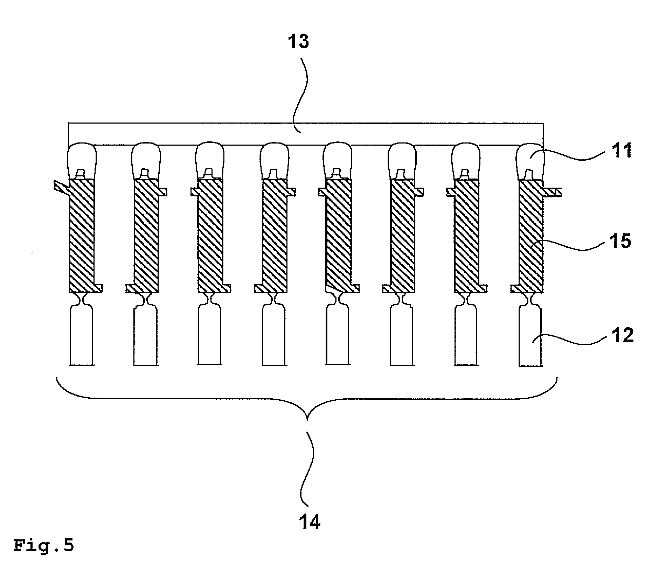

**Fig. 5** zeigt in einer vergrößerten Detailansicht die Vielzahl der Verabreichungsmittel (14) nach Trennung der flüssigkeitsdurchströmbaren Verbindungen zwischen den einzelnen Verabreichungsmitteln und der Trennung von der mindestens einen Zuführleitung (6 bzw. 8) und vom endständigen Aufnahmemittel (16). Die Füllung der Verabreichungsmittel mit dem gewünschten Blutprodukt ist schraffiert dargestellt. Es stehen jetzt die gefüllten Verabreichungsmittel mit aliquoten Anteilen des gewünschten Blutproduktes zur Applikation am Patienten bereit. Die Verabreichungsmittel können einzeln vom Verbindungssteg (13) entnommen werden, mit dem Öffnungsmittel (11) geöffnet werden und vom Patienten selber oder einer anderen Person dem erfindungsgemäßem Gebrauch zugeführt werden.

Geeignete Materialien für die erfindungsgemäße Vorrichtung sind beispielsweise aber nicht ausschließlich:
- Polyvinylchlorid (PVC), Polyurethan (PU, PUR)

Geeignete Entnahmemittel sind beispielsweise aber nicht ausschließlich:
- Transfusionskanülen, Hohlnadeln

Geeignete Mittel in zusätzlichen Behältern der Vorrichtung, die mit den Mitteln (1, 5 oder 10) flüssigkeitsdurchströmbar verbunden sind, um Arzneimittel aus Blut oder Blutprodukten herzustellen, sind beispielsweise aber nicht ausschließlich:
- Verdünnungslösungen (beispielsweise Kochsalzlösung), Antikoagulantien (beispielsweise EDTA), weitere pharmazeutisch akzeptable Substanzen (beispielsweise BSS^{®}, Balanced Salt Solution)

Geeignete Trennmittel zwischen den Mitteln 1, 5, 10) der Vorrichtung sind beispielsweise aber nicht ausschließlich:
- Schlingen, Knoten, Klemmen, Kugeln, Kegeln, Ventilen, verkapselten Abbrechteile, Knickbrechverbindungen, etc.

Geeignete Verfahren oder Werkzeuge zum sterilen Trennen von Kunststoffschläuchen oder -röhren etc. sind beispielsweise aber nicht ausschließlich:
- Trennschweißen, etwa mit quetschend-schneidenden Werkzeugen wie beispielsweise Zangen
- Thermoschweißen mit Sollrissstelle
- Einfrieren der beiden Enden jenseits der Trennstelle vor der Trennung
- Ultraschallschweißen

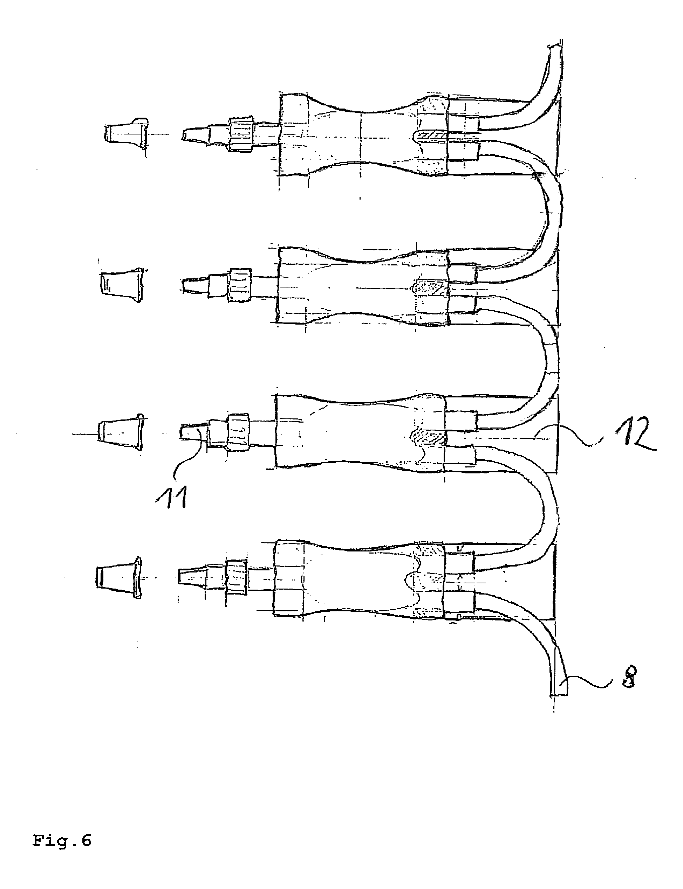

**Fig. 6** zeigt in einer Detailansicht eine ganz besonders vorteilhafte Anordnung der Vielzahl von Verabreichungsmitteln (14). Jedes einzelne Verabreichungsmittel weist einen Schraubverschluss auf, der besonders bevorzugt in Form eines Luer-Schraubverschlusses mit einer abnehmbaren Verschlusskappe ausgebildet ist. Die zuführenden und die abführenden flüssigkeitsdurchströmbaren Verbindungen zwischen den einzelnen Verabreichungsmitteln sind als Schlauchverbindungen ausgebildet und befinden sich gegenüber dem jeweiligen Schraubverschluss, um eine Verletzungsgefahr beim Tropfen in das Auge auszuschließen. Die Vielzahl der Verabreichungsmittel ist unabhängig von dem Entnahmemittel und den Sammel- und Trennmittel zu sehen. In einer bevorzugten Ausführungsform wird die Vielzahl von Verabreichungsmitteln an jeweils in der Größe passende Sammel- und Trennmittel durch eine Sterilschweissung verbunden, so dass je nach dem Bedarf an Augenserumtropfen eine passende Menge an Blut entnommen wird.

### Liste der Schritte des Verfahrens:

1) Nach Schaffung eines Zugangs zum venösen Blutgefäßsystem eines Blutspenders mit dem Entnahmemittel (1), insbesondere durch die dem Fachmann bekannte Punktion einer Vene, wird das Blut im mindestens einem Sammel- und Trennmittel (5) gesammelt.
   Dabei sind folgende Modifikationen möglich:
   - Zugabe von Verdünnungsmitteln, Mittel zur Hemmung der Blutgerinnung oder anderer pharmazeutisch akzeptabler Substanzen
   - Vorsehung dieser Mittel in der Vorrichtung.
   - Sterile Trennung des Entnahmemittels (1) vom Rest der erfindungsgemäßen Vorrichtung nach Beendigung der Blutentnahme.
   - Sterile Trennung des Sammelmittels zur Durchführung des *predonation samplings* (2) vom Rest der erfindungsgemäßen Vorrichtung nach Beendigung der Blutentnahme.
2) Trennung des Blutserums vom restlichen Blut durch Gerinnung und Bildung des Blutserums als Überstand über den restlichen Anteilen des Blutes im mindestens einem Sammel- und Trennmittel (5).
   Dabei sind folgende Modifikationen möglich:
   - Gerinnung bei Umgebungstemperatur und für über ca. 1 Stunde, vorzugsweise über 2 Stunden.
   - Besonders vorteilhaft ist die Beschleunigung und Verbesserung der Trennung des Blutserums von den restlichen Anteilen des Blutes durch Zentrifugieren der erfindungsgemäßen Vorrichtung, mindestens aber des Sammel- und Trennmittels (5).
3) Verbringung des gewonnenen Blutserums in die Vielzahl von Verabreichungsmitteln (10).
   Dabei sind folgende Modifikationen möglich:
   - Ggf. Verbringung durch eine Sterilfiltrationseinheit oder einen Leukozytenfilter oder einen Zellfilter.
   - Zugabe weiterer pharmazeutisch akzeptabler Substanzen oder Vorsehung weiterer pharmazeutisch akzeptabler Substanzen in der Vorrichtung.
4) Sterile Trennung der Vielzahl von Verabreichungsmitteln (14) vom Rest der erfindungsgemäßen Vorrichtung. Die Verabreichungsmittel stehen damit zur Verwendung am Patienten zur Verfügung.

Weitere Schritte können ggf. nachgeschaltet werden und umfassen die Aliquotierung bei Verwendung einer Vielzahl von Verabreichungsmitteln (14), die Kontrolle auf Sterilität (wird beispielsweise erleichtert durch die Vorsehung eines Sammelmittels zur Durchführung des *predonation samplings* (2)), die Lagerung der mit dem gewünschten Blutprodukt gefüllten Verabreichungsmittel beispielsweise mittels Gefrierlagerung bei rund -20° C oder die Lagerung zur Abgabe innerhalb von 12 bis 24 Stunden bei 4° C mit Verwerfung der Applikationsdosis bei Nichtnutzung im genannten Zeitraum. Ein Transport der mit dem gewünschten Blutprodukt gefüllten Verabreichungsmittel ist ebenfalls möglich.

### Bezugszeichenliste

(1) Entnahmemittel
(2) Sammelmittel zur Durchführung des *predonation samplings*
(5) Sammel- und Trennmittel
(6) Zuführleitung
(7) zusätzliches Sammel- und Trennmittel
(8) weiterführende Zuführleitung
(10) Verabreichungsmittel
(11) Öffnungsmittel
(12) Vorrichtung für eine Markierung
(13) Verbindungssteg
(14) Vielzahl der Verabreichungsmittel
(15) hinterstes Verabreichungsmittel
(16) endständiges Aufnahmemittel

## Patentansprüche

1. Vorrichtung zur Entnahme von Blut und Herstellung von Blutprodukten daraus und deren Lagerung und Transport, umfassend Entnahmemittel (1) und mindestens ein Sammel- und Trennmittel (5), **dadurch gekennzeichnet, dass** die Vorrichtung eine Vielzahl an Verabreichungsmitteln (10, 14) umfasst, wobei Entnahmemittel (1), Sammel- und Trennmittel (5) und die Verabreichungsmittel (10, 14) flüssigkeitsdurchströmbar miteinander verbunden sind und jedes Verabreichungsmittel (10, 14) ein Öffnungsmittel (11) aufweist, welches zur Öffnung durch einen Patienten oder eine andere Person ohne weitere Hilfsmittel geeignet ausgeführt ist.

2. Vorrichtung nach Anspruch 1, **dadurch gekennzeichnet, dass** Entnahmemittel (1), Sammel- und Trennmittel (5) und die Verabreichungsmittel (10) aus sterilisierbarem Material, wie vorzugsweise Polyvinylchlorid (PVC) oder Polyurethan (PU, PUR) bestehen und entweder einstückig ausgeführt sind oder aus mehreren Teilelementen bestehen.

3. Vorrichtung nach Anspruch 1 bis 2, **dadurch gekennzeichnet, dass** Entnahmemittel (1), Sammel- und Trennmittel (5) und die Verabreichungsmittel (10) so ausgeführt sind, dass eine Trennung mindestens der Verabreichungsmittel (10) vom Rest der Vorrichtung ohne Verlust der Sterilität der sich in der Vorrichtung, mindestens jedoch der sich in dem mindestens einen Verabreichungsmittel (10) befindlichen Blutproduktes möglich ist.

4. Vorrichtung nach Anspruch 1 bis 3, **dadurch gekennzeichnet, dass** die Verabreichungsmittel (10 bzw. 14) ein Volumen von bis zu 400 ml aufweisen, bevorzugt ein Volumen von bis zu 100 ml aufweisen, besonders bevorzugt ein Volumen von bis zu 10 ml aufweisen und ganz besonders bevorzugt ein Volumen von weniger als 10 ml pro Verabreichungsmittel aufweisen.

5. Vorrichtung nach Anspruch 1 bis 4, **dadurch gekennzeichnet, dass** zwischen dem Sammel- und Trennmittel (5) und den Verabreichungsmitteln (10, 14) ein zusätzliches Sammel- und Trennmittel (7) vorgesehen ist, welches zur Trennung der Blutbestandteile voneinander und zur Weiterleitung der einzelnen Blutbestandteile geeignet ausgeführt ist.

6. Vorrichtung nach Anspruch 1 bis 5, **dadurch gekennzeichnet, dass** mindestens das Sammel- und Trennmittel (5) oder die gesamte Vorrichtung so ausgeführt und aus solchem Material, vorzugsweise Kunststoff, wie Polyvinylchlorid (PVC) oder Polyurethan (PU, PUR) ausgeführt ist, dass das Zentrifugieren des Inhalts ohne Aufhebung der Sterilität dieses Inhalts, d.h. im Wesentlichen ohne Beschädigung der Wandung des Sammel- und Trennmittels (5) oder der gesamten Vorrichtung möglich ist.

7. Vorrichtung nach Anspruch 1 bis 6, **dadurch gekennzeichnet, dass** die Verabreichungsmittel (14) entweder seriell und dann alternierend jeweils oben und/oder unten, in Bezug auf die Behältnisform der vorzugsweise gestreckt ausgeführten Verabreichungsmittel (10, 14), von dem Inhalt aus dem Sammel- und Trennmittel (5) anströmbar ausgeführt sind oder parallel anströmbar ausgeführt sind.

8. Vorrichtung nach Anspruch 1 bis 7, **dadurch gekennzeichnet, dass**: in Strömungsrichtung bei Verbringung des Inhaltes aus dem Sammel- und Trennmittel (5) oder dem zusätzlichen Sammel- und Trennmittel (7) in die Verabreichungsmittel (10) jenseits der Verabreichungsmittel (10) und mit diesen flüssigkeitsverbunden ein endständiges Aufnahmemittel (16) zur Aufnahme von Luft oder/und überschüssigem Inhalt vorgesehen ist, oder die Vielzahl von Verabreichungsmitteln (14) seriell angeströmt sind und nach dem, in Strömungsrichtung, hinterstem Verabreichungsmittel (15) ein endständiges Aufnahmemittel (16) zur Aufnahme von Luft oder/und überschüssigem Inhalt vorgesehen ist.

9. Vorrichtung nach Anspruch 1 bis 8, **dadurch gekennzeichnet, dass** an dem Entnahmemittel (1) ein weiteres Sammelmittel zur Durchführung des *predonation samplings* (2), vorzugsweise in Form eines *predonation sampling bags* vorgesehen ist.

10. Vorrichtung nach Anspruch 1 bis 9, **dadurch gekennzeichnet, dass** zwischen den flüssigkeitsdurchströmbaren Elementen der Vorrichtung, mindestens jedoch zwischen dem Sammel- und Trennmittel (5) und den Verabreichungsmitteln (10) mindestens einmal aktivier- und deaktivierbare Trennmittel, vorzugsweise Knickbrechverbindungen vorgesehen sind.

11. Vorrichtung nach Anspruch 1 bis 10, **dadurch gekennzeichnet, dass** auf beiden Seiten jenseits einer vorgesehenen Trennstelle zwischen Entnahmemittel (1) und dem Sammelmittel zur Durchführung des *predonation samplings* (2), sowie zwischen Sammel- und Trennmittel (5) und den Verabreichungsmitteln (10) an jedem dieser Elemente mindestens ein Dokumentationsfeld vorgesehen oder bereits gekennzeichnet vorgesehen ist, etwa durch Einprägung an den Wandungen der Mittel (2, 5, 10, 14, 15), bevorzugt in Form einer fortlaufenden Seriennummer, besonders bevorzugt als Barcode, um eine nachträgliche Zuordnung des Blutspenders zu den Verabreichungsmitteln (10) zu ermöglichen.

12. Vorrichtung nach Anspruch 1 bis 11, **dadurch gekennzeichnet, dass** sie mit Mitteln zur Herstellung eines Arzneimittels aus dem Spenderblut in weiteren Behältnisse vorgesehen ist, etwa zum Zwecke der Verdünnung oder der Verhinderung der Blutgerinnung oder des Einsatzes weiterer pharmakologisch akzeptabler Substanzen, welche flüssigkeitsdurchströmbar mit den Mitteln (1, 5, 10) der Vorrichtung verbunden sind oder die Mittel (1, 5, 10) mit solchen Mitteln vorbefüllt ausgeführt sind.

13. Verfahren zur Herstellung von Blutbestandteilen in einer Vorrichtung gemäß Anspruch 1, **gekennzeichnet durch** die folgenden Schritte:
1. Sammlung von Blut im Sammel- und Trennmittel (5).
2. Trennung des Blutes in mindestens ein Blutprodukt und die restlichen Blutbestandteile im Sammel- und Trennmittel (5).
3. Verbringung des mindestens einen Blutproduktes in eine Vielzahl von Verabreichungsmitteln (10).
4. Sterile Trennung der Vielzahl von Verabreichungsmitteln (10) vom Rest der Vorrichtung.

14. Verfahren nach Anspruch 13, **dadurch gekennzeichnet, dass** das mindestens eine Blutprodukt durch eine Sterilfiltrationseinheit oder einen Leukozytenfilter oder einen anderen Zellfilter verbracht wird.

15. Verfahren nach Anspruch 13 und 14, **dadurch gekennzeichnet, dass** die Verbringung des mindestens einen Blutproduktes durch eine Sterilfiltrationseinheit oder einen Leukozytenfilter oder einen anderen Zellfilter nach dem Sammel- und Trennmittel (5) erfolgt.

16. Verfahren nach Anspruch 13 bis 15, **dadurch gekennzeichnet, dass** das mindestens eine Blutprodukt Serum ist und die Trennung des Blutes durch Gerinnung und Bildung des Serums als Überstand über den restlichen Anteilen des Blutes erfolgt.

17. Verfahren nach Anspruch 13 bis 16, **dadurch gekennzeichnet, dass** die Trennung der Blutprodukte durch Zentrifugieren der Vorrichtung gemäß Anspruch 1 oder des Sammel- und Trennmittels (5) erfolgt.

## Claims

1. Device for withdrawing blood and producing blood products therefrom and for storing and transporting said products, comprising withdrawal means (1) and at least one collection and separation means (5), **characterised in that** the device comprises a plurality of administration means (10, 14), the withdrawal means (1), collection and separation means (5) and administration means (10, 14) being interconnected such that fluid can flow therethrough and each administration means (10, 14) comprising an opening means (11) which is suitably designed to be opened by a patient or another person without the need for additional aids.

2. Device according to claim 1, **characterised in that** the withdrawal means (1), collection and separation means (5) and administration means (10) consist of sterilisable material, preferably polyvinyl chloride (PVC) or polyurethane (PU, PUR) for example, and are either integrally formed or consist of a plurality of subelements.

3. Device according to claims 1 and 2, **characterised in that** the withdrawal means (1), collection and separation means (5) and administration means (10) are designed such that it is possible to separate at least the administration means (10) from the rest of the device without loss of sterility of the blood product in the device, or at least of the blood product in the at least one administration means (10).

4. Device according to claims 1 to 3, **characterised in that** the administration means (10 and 14) have a volume of up to 400 ml, preferably a volume of up to 100 ml, particularly preferably a volume of up to 10 ml and more preferably a volume of less than 10 ml per administration means.

5. Device according to claims 1 to 4, **characterised in that** an additional collection and separation means (7) is provided between the collection and separation means (5) and the administration means (10, 14) and is suitably designed to separate the blood components from one another and transfer the individual blood components.

6. Device according to claims 1 to 5, **characterised in that** at least the collection and separation means (5) or the entire device is designed in such a way and from such a material, preferably a plastics material such as polyvinyl chloride (PVC) or polyurethane (PU, PUR), that it is possible to centrifuge the contents without loss of sterility of said contents, i.e. essentially without damaging the wall of the collection and separation means (5) or the entire device.

7. Device according to claims 1 to 6, **characterised in that** the administration means (14) are designed such that the contents of the collection and separation means (5) can flow therethrough either in series, and then in an alternating fashion from above and/or below, in each case, depending on the type of container of the preferably elongate administration means (10, 14), or in parallel.

8. Device according to claims 1 to 7, **characterised in that**, when the contents are passed from the collection and separation means (5) or from the additional collection and separation means (7) to the administration means (10), a terminal holding means (16) for holding air and/or surplus contents is provided downstream of the administration means (10) in the flow direction and is in fluidic communication therewith, or fluid flows through the plurality of administration means (14) in series and a terminal holding means (16) for holding air and/or surplus contents is provided downstream of the rearmost administration means (15) in the flow direction.

9. Device according to claims 1 to 8, **characterised in that** an additional collection means for carrying out the *predonation sampling* (2), preferably in the form of a *predonation sampling bag,* is provided on the withdrawal means (1).

10. Device according to claims 1 to 9, **characterised in that** separation means, preferably bend/break connections, which can be activated and deactivated at least once, are provided between the elements of the device through which fluid can flow, or at least between the collection and separation means (5) and the administration means (10).

11. Device according to claims 1 to 10, **characterised in that** at least one documentation field is provided or is already labeled on each of said elements on both sides beyond a separation point provided between the withdrawal means (1) and the collection means for carrying out the *predonation sampling* (2), and between the collection and separation means (5) and the administration means (10), for example by means of impressing on the walls of the means (2, 5, 10, 14, 15), preferably in the form of a consecutive serial number, particularly preferably in the form of a barcode, so as to make it possible to subsequently assign the blood donor to the administration means (10).

12. Device according to claims 1 to 11, **characterised in that** said device is provided with means for producing a pharmaceutical product from the donated blood in additional containers, for example for thinning or preventing blood coagulation or for using additional pharmacologically acceptable substances, which means are connected to the means (1, 5, 10) of the device such that fluid can flow therethrough, or the means (1, 5, 10) are designed to be pre-filled with such products.

13. Method for producing blood components in a device according to claim 1, **characterised by** the steps of:
1. collecting blood in the collection and separation means (5),
2. separating the blood in the collection and separation means (5) into at least one blood product and the residual blood components,
3. passing the at least one blood product into a plurality of administration means (10),
4. separating the plurality of administration means (10) from the rest of the device in a sterile manner.

14. Method according to claim 13, **characterised in that** the at least one blood product is passed through a sterile filtration unit or a leukocyte filter or another cell filter.

15. Method according to claims 13 and 14, **characterised in that** the at least one blood product is passed through a sterile filtration unit or a leukocyte filter or another cell filter downstream of the collection and separation means (5).

16. Method according to claims 13 to 15, **characterised in that** the at least one blood product is serum and the blood is separated by coagulation and the formation of the serum as the supernatant above the residual fractions of the blood.

17. Method according to claims 13 to 16, **characterised in that** the blood products are separated by centrifuging the device according to claim 1 or the collection and separation means (5).

## Revendications

1. Dispositif pour le prélèvement de sang et la fabrication de produits sanguins à partir de celui-ci ainsi que pour leur stockage et leur transport, comprenant un moyen de prélèvement (1) et au moins un moyen de collecte et de séparation (5), **caractérisé en ce que** le dispositif comprend une pluralité de moyens d'administration (10, 14), dans lequel le moyen de prélèvement (1), le moyen de collecte et de séparation (5) et les moyens d'administration (10, 14) sont reliés l'un à l'autre par communication fluidique et chaque moyen d'administration (10, 14) présente un moyen d'ouverture (11) qui est conçu pour convenir à l'ouverture par un patient ou une autre personne sans autres moyens auxiliaires.

2. Dispositif selon la revendication 1, **caractérisé en ce que** le moyen de prélèvement (1), le moyen de collecte et de séparation (5) et les moyens d'administration (10) sont constitués d'un matériau stérilisable, de préférence de poly(chlorure de vinyle) (PVC) ou de polyuréthane (PU, PUR) et formés d'un seul tenant ou de plusieurs éléments partiels.

3. Dispositif selon les revendications 1 et 2, **caractérisé en ce que** le moyen de prélèvement (1), le moyen de collecte et de séparation (5) et les moyens d'administration (10) sont conçus de manière que soit possible une séparation au moins du moyen d'administration (10) du restant du dispositif sans perte de stérilité du produit sanguin se trouvant dans le dispositif, mais au moins dans le au moins un moyen d'administration (10).

4. Dispositif selon les revendications 1 à 3, **caractérisé en ce que** les moyens d'administration (10 ou 14) présentent un volume allant jusqu'à 400 ml, de préférence un volume allant jusqu'à 100 ml, en particulier un volume allant jusqu'à 10 ml et tout particulièrement un volume de moins de 10 ml par moyen d'administration.

5. Dispositif selon les revendications 1 à 4, **caractérisé en ce qu'**il est prévu entre le moyen de collecte et de séparation (5) et les moyens d'administration (10, 14) un moyen supplémentaire de collecte et de séparation (7) qui est conçu de manière appropriée pour séparer les composants sanguins l'un de l'autre et transmettre les composants sanguins individuels.

6. Dispositif selon les revendications 1 à 5, **caractérisé en ce qu'**au moins le moyen de collecte et de séparation (5) ou le dispositif dans son ensemble est conçu et formé d'un matériau, de préférence une matière plastique, telle que le poly(chlorure de vinyle) (PVC) ou le polyuréthane (PU, PUR) de sorte que la centrifugation du contenu soit possible sans renoncer à la stérilité de ce contenu, c'est-à-dire sensiblement sans porter atteinte à la paroi du moyen de collecte et de séparation (5) ou à la totalité du dispositif.

7. Dispositif selon les revendications 1 à 6, **caractérisé en ce que** les moyens d'administration (14) sont conçus de manière à pouvoir être alimentés en série et ensuite en alternance, respectivement, par le haut et/ou par le bas, par rapport à la forme de récipient des moyens d'administration conformés de préférence allongés (10, 14) ou en parallèle par le contenu du moyen de collecte et de séparation (5).

8. Dispositif selon les revendications 1 à 7, **caractérisé en ce qu'**il est prévu, dans la direction d'écoulement dans l'acheminement du contenu du moyen de collecte et de séparation (5) ou du moyen de collecte et de séparation supplémentaire (7) dans les moyens d'administration (10), de l'autre côté des moyens d'administration (10) et en communication fluidique avec ceux-ci, un moyen de réception terminal (16) pour recevoir de l'air et/ou un contenu excédentaire ou bien la pluralité de moyens d'administration (14) sont alimentés en série et il est prévu, après le moyen d'administration (15) le plus en arrière dans la direction d'écoulement, un moyen de réception terminal (16) pour recevoir de l'air et/ou un contenu excédentaire.

9. Dispositif selon les revendications 1 à 8, **caractérisé en ce qu'** il est prévu sur le moyen de prélèvement (1) un autre moyen de collecte pour réaliser l'échantillonnage de pré-don (2), de préférence sous la forme d'un sachet d'échantillonnage de pré-don.

10. Dispositif selon les revendications 1 à 9, **caractérisé en ce qu'**il est prévu entre les éléments du dispositif traversables par les liquides, mais au moins entre le dispositif de collecte et de séparation (5) et les moyens d'administration (10), au moins un moyen de séparation à la fois activable et désactivable, de préférence des liaisons frangibles par coudage.

11. Dispositif selon les revendications 1 à 10, **caractérisé en ce qu'**il est prévu sur les deux côtés respectivement d'une interface prévue entre le moyen de prélèvement (1) et le moyen de collecte pour réaliser l'échantillonnage de pré-don (2) ainsi qu'entre le moyen de collecte et de séparation (5) et les moyens d'administration (10), sur chacun de ces éléments, au moins un champ de documentation ou il est déjà prévu de manière caractérisée notamment par impression sur les parois des moyens (2, 5, 10, 14, 15), de préférence sous la forme d'un numéro de série en continu, tout particulièrement sous la forme d'un code à barres, pour permettre une affectation ultérieure du donneur de sang aux moyens d'administration (10).

12. Dispositif selon les revendications 1 à 11, **caractérisé en ce qu'**il est pourvu de moyens de fabrication d'un médicament à partir du sang du donneur dans d'autres récipients notamment à des fins de dilution ou pour empêcher la coagulation du sang ou encore pour ajouter d'autres substances acceptables au plan pharmacologique, qui sont reliés en communication fluidique avec les moyens (1, 5, 10) du dispositif ou les moyens (1, 5, 10) sont conçus pour être pré-remplis par ces agents.

13. Procédé de fabrication de composants sanguins dans un dispositif selon la revendication 1, **caractérisé par** les étapes consistant à :
1. recueillir le sang dans le moyen de collecte et de séparation (5) ;
2. séparer le sang en au moins un produit sanguin et en composants sanguins restants dans le moyen de collecte et de séparation (5) ;
3. acheminer le au moins un produit sanguin dans une pluralité de moyens d'administration (10) ; et
4. séparer de manière stérile la pluralité de moyens d'administration (10) du restant du dispositif.

14. Procédé selon la revendication 13, **caractérisé en ce que** le au moins un produit sanguin est acheminé à travers une unité de filtration stérile ou un filtre à leucocytes ou un autre filtre cellulaire.

15. Procédé selon les revendications 13 et 14, **caractérisé en ce que** l'acheminement du au moins un produit sanguin à travers une unité de filtration stérile ou un filtre à leucocytes ou un autre filtre cellulaire se fait après le moyen de collecte et de séparation (5).

16. Procédé selon les revendications 13 à 15, **caractérisé en ce que** le au moins un produit sanguin est du sérum et la séparation du sang par coagulation et formation du sérum comme surnageant se fait sur les parties restantes du sang.

17. Procédé selon les revendications 13 à 16, **caractérisé en ce que** la séparation des produits sanguins se fait par centrifugation du dispositif selon la revendication 1 ou du moyen de collecte et de séparation (5).
